# EUROPEAN PATENT SPECIFICATION

(11) **EP 2 792 375 B1**
(45) Date of publication and mention of the grant of the patent: **04.11.2015**
(21) Application number: 14161830.6
(22) Date of filing: 26.03.2014
(51) Int. Cl.: A61L 27/54, A61L 31/14, A61L 31/16, C08L 5/08

(54) **Medical device comprising a chitosan-based support structure**
Medizinische Vorrichtung mit Chitosan-Unterstützungsstruktur
Dispositif médical avec une structure de support de chitosane

(30) Priority: 19.04.2013 IT TO20130317
(43) Date of publication of application: 22.10.2014
(73) Proprietor: ARCHIMEDES S.r.l., 35123 Padova (PD) (IT)
(72) Inventor: ORNAGHI, Oscar Marco, 35031 Abano Terme (Padova) (IT); BINOTTO, Radames, 35031 Abano Terme (Padova) (IT); BINOTTO, Renzo, 35031 Abano Terme (Padova) (IT); VALLANA, Valerio, 35031 Abano Terme (Padova) (IT)
(74) Representative: Freyria Fava, Cristina

(56) References cited:
- WO-A1-2013/091662
- WO-A2-2004/105737
- US-A1- 2009 252 700
- US-A1- 2013 095 187
- US-B2- 8 062 669
- VALDATTA L ET AL: "Evaluation of the efficacy of polydeoxyribonucleotides in the healing process of autologous skin graft donor sites: A pilot study", CURRENT MEDICAL RESEARCH AND OPINION, INFORMA HEALTHCARE, GB, vol. 20, no. 3, 1 March 2004 (2004-03-01), pages 403-408, XP008122537, ISSN: 0300-7995

## Description

### FIELD OF THE INVENTION

The present description relates to a medical device comprising a chitosan-based carrying structure capable of preventing the formation of adhesions between tissues during regrowth.

### TECHNOLOGICAL BACKGROUND

"Adhesions" are bundles of fibrous tissue that form between tissues as a result of traumatic events, surgical wounds, infections or as a result of any condition that can trigger tissue repair.

Adhesions form in the same way that scars form and are constituted by the same type of tissue. The term "adhesion" is used because the fibrous tissue joins anatomical structures that are normally separate.

Adhesions can have particularly severe clinical consequences, especially in organs for which gliding of the tissue is necessary: for example, adhesions can cause tendon-gliding defects that alter joint function.

In addition, adhesions may cause compression of blood vessels and nerves with the consequent onset of major pain syndromes.

Substances and devices capable of limiting the formation of adhesions are of great interest in the field of medicine.

The solution commonly adopted for preventing the formation of adhesions is to insert barriers in the form of networks, films, membranes or gels into the area of the body where separation of tissues is required.

The limitation of this technique lies in the passivity of the intervention. Moreover, in the case of non-resorbable barriers, it is necessary to use coatings with materials that guarantee non-stick properties, such as polytetrafluoroethylene or similar, which do not always guarantee optimum results.

Furthermore, such non-resorbable barriers have the disadvantage of requiring removal through a further surgery once the tissue healing process is completed.

Resorbable barriers usually have the disadvantage of excessively rapid degradation with respect to the time required for reconstitution of the biological tissues. Additionally, their degradation is accompanied by inflammation caused by biological and chemical processes of resorption and elimination of the compounds derived from degradation of the barrier.

### SUMMARY OF THE INVENTION

The object of the present description is to provide a resorbable medical device capable of contrasting the formation of adhesions and at the same time actively promoting natural physiological regeneration of the tissue and contrasting inflammation.

In accordance with the invention, the above object is achieved by means of the solution specifically recalled in the annexed claims, which constitute an integral part of the present description.

An embodiment of the present description relates to a medical device comprising nanoparticles of chitosan and polydeoxyribonucleotides (PDRN) on a chitosan-based laminar carrying structure.

In a preferred embodiment, the carrying structure further comprises polyethylene oxide.

In a preferred embodiment, the carrying structure is in the form of nanofibers.

### BRIEF DESCRIPTION OF DRAWINGS

The invention will now be described in detail, by way of illustrative and non-limiting example only, with reference to the attached drawings, in which:
- **Figure 1** is a perspective view of one embodiment of a medical device according to the present description;
- **Figure 2** is a diagram of an electrospinning apparatus for the production of nanofibers;
- **Figure 3** is a scanning electron microscopy image of nanofibers of chitosan 70/1000 and polyethylene oxide obtained through an electrospinning process (magnification 6000x);
- **Figure 4** is a scanning electron microscopy image of nanofibers of chitosan 70/1000 and polyethylene oxide obtained through an electrospinning process (magnification 30000x);
- **Figure 5** shows the FTIR spectra of the components used in the production of carrying structures based on nanofibers of chitosan 70/1000 and polyethylene oxide (95:5);
- **Figure 6** shows the FTIR spectra of the components used in the production of carrying structures based on nanofibers of chitosan MMW and polyethylene oxide (95:5);
- **Figure 7** shows the TGA thermograms of carrying structures based on nanofibers of chitosan and polyethylene oxide subjected to cross-linking and not subjected to cross-linking;
- **Figure 8** shows the particle size distribution of particles of chitosan incorporating PDRN obtained by a process of coacervation of the chitosan-PDRN system. Dispersion index 0.307. In the ordinate the percent intensity and on the abscissa particle diameter in nm (solution 1 shown in Table 1);
- **Figure 9** shows the particle size distribution of chitosan particles incorporating PDRN obtained by a process of coacervation of the chitosan-PDRN system. Dispersion index 0.279. In the ordinate the percent intensity and on the abscissa particle diameter in nm (solution 2 shown in Table 1);
- **Figure 10** is a transmission electron microscopy image of nanoparticles obtained by a process of coacervation of the chitosan-PDRN system;
- **Figure 11** shows the particle size distribution of chitosan particles incorporating PDRN obtained by a process of coacervation of the chitosan-PDRN system. Dispersion index 0.165. In the ordinate the percent intensity and on the abscissa particle diameter in nm (solution 10 shown in Table 3);
- **Figure 12** is a transmission electron microscopy image of chitosan nanoparticles incorporating PDRN in the form of a sodium salt (PDRN-Na) obtained by an ionic gelation process;
- **Figure 13** is a UV spectrum of a sample of chitosan nanoparticles incorporating PDRN-Na obtained with the process of ionic gelation;
- **Figure 14** is a scanning electron microscopy image of nanoparticles deposited on a chitosan-based carrying structure by electrospraying (magnification 30000x).

### DETAILED DESCRIPTION OF EMBODIMENTS

In the following description, numerous specific details are provided to allow a complete understanding of the embodiments. The embodiments may be practiced without one or more of the specific details, or with other methods, components, materials, etc. In other instances, well-known structures, materials, or operations are not shown or described in detail to avoid obscuring aspects of the embodiments.

Reference throughout this specification to "one embodiment" or "an embodiment" means that a feature, structure, or characteristic described in relation to an embodiment is included in at least one embodiment. Therefore, the appearances of the terms "in one embodiment" or "in an embodiment" appearing in various places throughout the present description do not necessarily all refer to the same embodiment. Furthermore, the particular features, structures, or characteristics may be combined in any suitable way in one or more embodiments.

The headings provided here are for convenience only and do not interpret the scope or meaning of the embodiments.

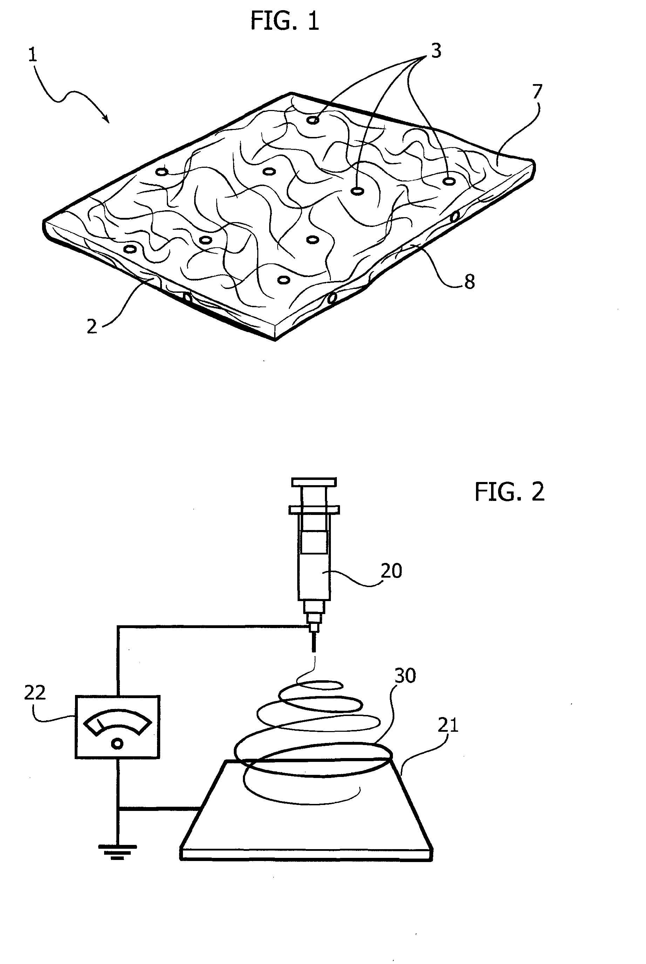

Figure 1 shows one embodiment of the medical device object of the present description, indicated by the reference 1.

In one embodiment, the medical device 1 comprises a laminar chitosan-based carrying structure 2, also called structure with a predominantly superficial extension. On one or both of its surfaces 7 and 8, the carrying structure 2 comprises chitosan-based particles 3 containing polydeoxyribonucleotides (PDRN).

In one embodiment, the carrying structure 2 further comprises polyethylene oxide.

In one embodiment, the carrying structure 2 is in the form of nanofibers of chitosan or a mixture of chitosan and polyethylene oxide.

Nanofibers of chitosan or chitosan and polyethylene oxide can be obtained using electrospinning methods known in the art (Homayoni et al., 2009).

In one embodiment, the carrying structure 2 has a thickness comprised between 80 and 160 µm, preferably between 100 and 120 µm.

In one embodiment, the nanofibers comprise chitosan and polyethylene oxide in a weight ratio comprised between 80:20 and 100:0, preferably equal to 95:5.

In one embodiment, the nanofibers have a diameter between 50 and 200 nm, preferably equal to 100 nm.

In one embodiment, the nanofibers are cross-linked by means of a cross-linking process that involves exposing it to glutaraldehyde vapors.

In one embodiment, the chitosan nanoparticles incorporate polydeoxyribonucleotides (PDRN) internally.

In one embodiment, the nanoparticles have a diameter comprised between 150-250 nm, preferably equal to about 200 nm.

In one embodiment, the nanoparticles comprise chitosan and polydeoxyribonucleotides in a weight ratio comprised between 3:1 and 5:1, preferably equal to 4:1.

The polydeoxyribonucleotides (PDRN) promote the growth of cells that are active in tissue regeneration, by stimulating the metabolism of fibroblasts as well as the production of dermal matrix components.

PDRN also promote angiogenesis in the treatment of wounds and burns, to accelerate the healing of skin micro-lesions and the production of cytokines and growth factors.

In one embodiment, PDRN may be employed in the form of salts such as salts of sodium, iron, chromium, magnesium, manganese and others.

Release of the PDRN from chitosan particles takes place for example through enzymatic degradation and/or swelling of the chitosan in the presence of extracellular fluids.

The examples below are intended to provide indications for implementing the device of the present description. However, these examples are non-limiting, as the device object of the present description can also be practiced with other methods known to the person skilled in the art.

### Production of carrying structures of chitosan nanofibers

In order to obtain carrying structures of electrospun chitosan nanofibers, two solutions of chitosan dissolved in acetic acid (90% in aqueous solution) were prepared:
- the first solution comprises chitosan 70/1000 (HeppeMedicalChitosan GmbH) with a *Deacetylation Degree* (DD) equal to 70% and a viscosity of 1000 cps (1.6% solution by weight in acetic acid);
- the second solution comprises chitosan MMW (Sigma-Aldrich) with a molecular weight between 500 and 1000 kDa, a DD% between 75 and 85% and a viscosity between 200 and 800 cps (1.6% solution by weight in acetic acid).

To each of the two solutions an additional polymer was added, namely polyethylene oxide (PEO) (1.6% solution by weight in acetic acid) having a molecular weight of 900 kDa.

In particular, two final solutions were obtained:
- a first solution comprising chitosan 70/1000-PEO with a weight ratio of 95:5;
- a second solution comprising chitosan MMW-PEO with a weight ratio of 95:5.

The two solutions comprising chitosan, acetic acid and PEO were subjected to slow, continuous mechanical stirring at ambient temperature for several hours to ensure complete dissolution of the polymers.

The two solutions were then subjected to electrospinning using an apparatus shown schematically in Figure 2, consisting of:
- a pump (not shown), on which a syringe 20 containing the polymer solution to electrospin is loaded,
- an electric generator 22 capable of generating negative and positive electrical potentials with voltages comprised between 0 and 100 kV and between -100 and 0 kV, respectively, and
- a metal collector 21 to collect the nanofibers 30.

The syringe 20 has a volume of about 5 cm³ and is equipped with a flat tipped metal needle.

The diameter of the needles is comprised between 27G and 22G (the diameter is chosen according to the viscosity of the polymer solution to be spun).

The metal collector 21 is covered by a sheet of aluminum (not shown) on which the nanofibers 30 of chitosan-PEO produced by electrospinning are deposited.

Electrospinning of the above mentioned chitosan solutions was performed using a potential of 18-20kV, a distance between the syringe needle and collector of 10 cm, and a solution flow rate of 0.8 ml/h.

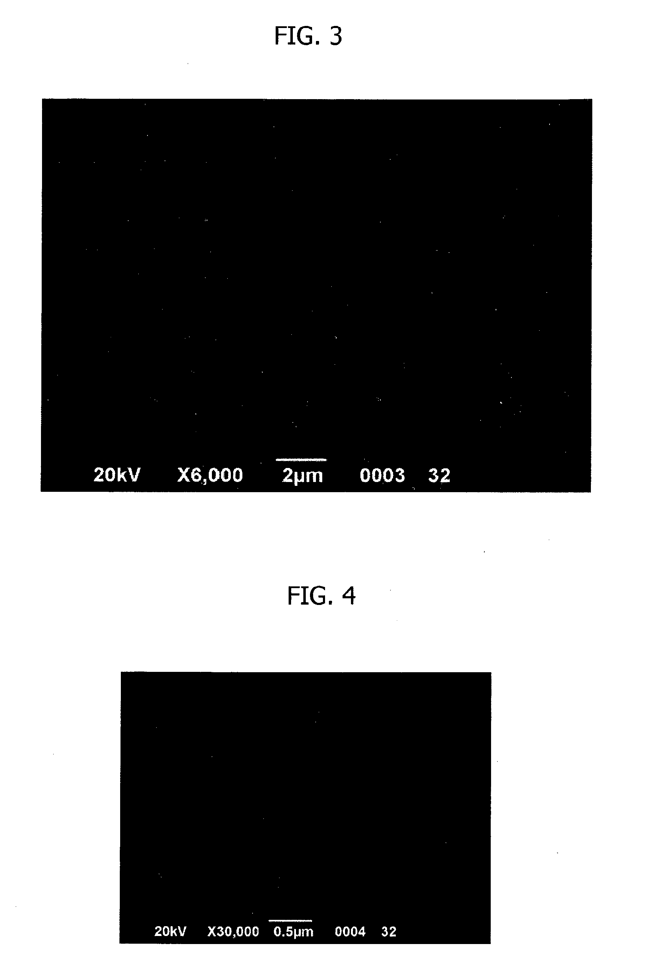

Figure 3 and Figure 4 show scanning electron microscopy images of nanofibers obtained with the electrospinning procedure, which provided carrying structures with a thickness of about 100-120 µm.

Some nanofiber samples obtained with the electrospinning process were subjected to cross-linking by exposure to vapors of glutaraldehyde (25% aqueous solution, Sigma Aldrich) to create intermolecular bonds between the fibers and make the carrying structures more stable.

Cross-linked and non-cross-linked carrying structures were analyzed using:
- Fourier transform infrared spectroscopy (FTIR);
- thermogravimetric (TGA) analysis;
- enzymatic degradation with lysozyme and analysis of swelling.

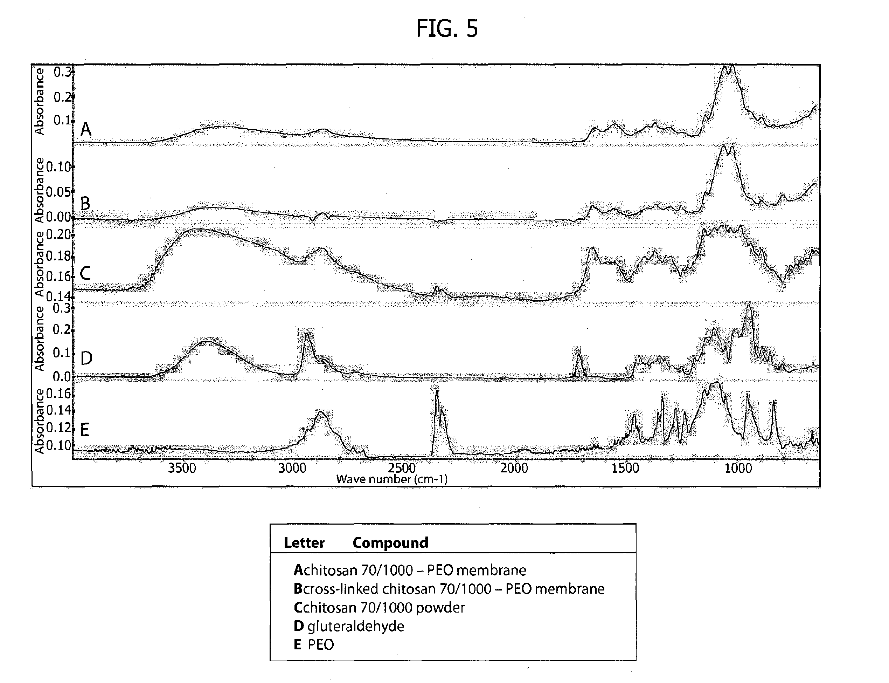

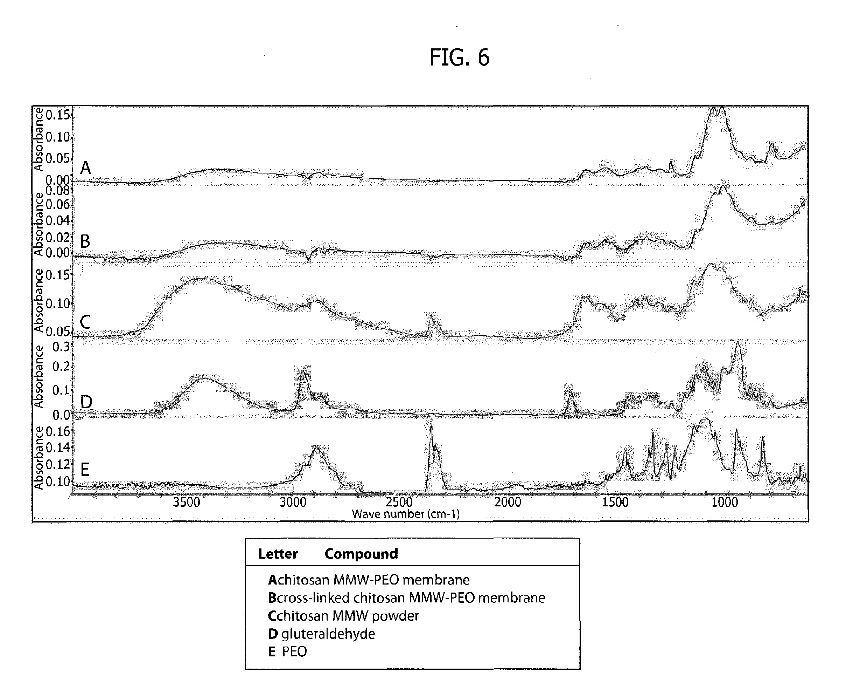

FTIR chemical characterization of the carrying structures of cross-linked and non-cross-linked nanofibers of chitosan 70/1000-PEO (95:5) (Figure 5) and carrying structures of cross-linked and non-cross-linked nanofibers of chitosan MMW-PEO (95:5) (Figure 6) reveal spectra that are consistent with characteristic spectra of the materials used, and do not show a difference between cross-linked and non-cross-linked carrying structures.

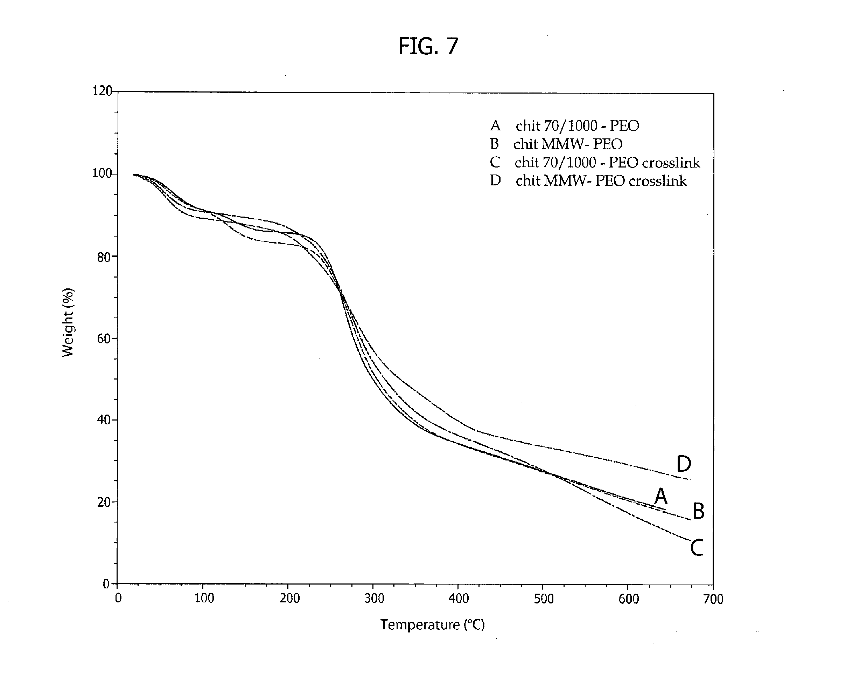

Thermogravimetric analysis (TGA) (Figure 7) showed that the most effective cross-linking, i.e., a greater number of intermolecular bonds between fibers, is obtained with nanofibers of MMW chitosan/PEO (95:5) compared to nanofibers of chitosan 70/1000 -PEO (95:5).

Assessment of enzymatic degradation described above is achieved by immersing the carrying structures in a buffered aqueous solution (with the enzyme lysozyme) and monitoring variations in weight at regular intervals of a few days or weeks.

The structure is removed and the aqueous solution absorbed with absorbent paper, then the structure is weighed.

Analysis of swelling and enzymatic degradation of the carrying structure revealed that the amount of water absorbed by the structure over a period of 48 h is about 20% by weight, whereas degradation was monitored over the course of 60 days.

The enzymatic degradation results show that the structure retains its barrier characteristics for at least 1 month and this time can be modulated via cross-linking.

### Production of nanoparticles of chitosan and PDRN through coacervation of a chitosan-PDRN system

Two solutions are initially prepared for the production of nanoparticles of chitosan and PDRN according to the coacervation method (Mao et al., 2001; Mohammadi et al., 2011):
- a first solution containing chitosan LWM (molecular weight 140-220 kDa, Sigma Aldrich) (0.02% by weight) dissolved in 5 mM CH₃COONa buffer, pH 5.4;
- a second solution containing PDRN (50 µg/ml) dissolved in 25 mM Na₂SO₄.

The two solutions are heated to 50-55°C.

The chitosan solution is added "dropwise" to the second solution with rapid stirring for 15-30 seconds to obtain a final solution.

Table 1 shows the amount of chitosan, PDRN, CH₃COONa and Na₂SO₄ in the two final solutions (solutions 1 and 2) prepared as indicated above and subjected to Light Scattering analysis to determine the size of the particles obtained.

**Table 1**

| **Sol.** | **Chitos %** | **CH₃COONa buffer (mM)** | **V_{chit} (ml)** | **PDRN (µg/ml)** | **Na₂SO₄ (mM)** | **V_{PDRN} (ml)** | **N/P** |
|---|---|---|---|---|---|---|---|
| **1** | 0.02 | 5 | 1 | 50 | 25 | 1 | 4/1 |
| **2** | 0.02 | 5 | 1 | 50 | 25 | 1 | 4/1 |

| | | | | | | | |
|---|---|---|---|---|---|---|---|
| N /P indicates the Chitosan/PDRN ratio | | | | | | | |

The final solutions 1 and 2, containing nanoparticles were filtered through a syringe filter with a 0.45 µm pore diameter to eliminate any insoluble material.

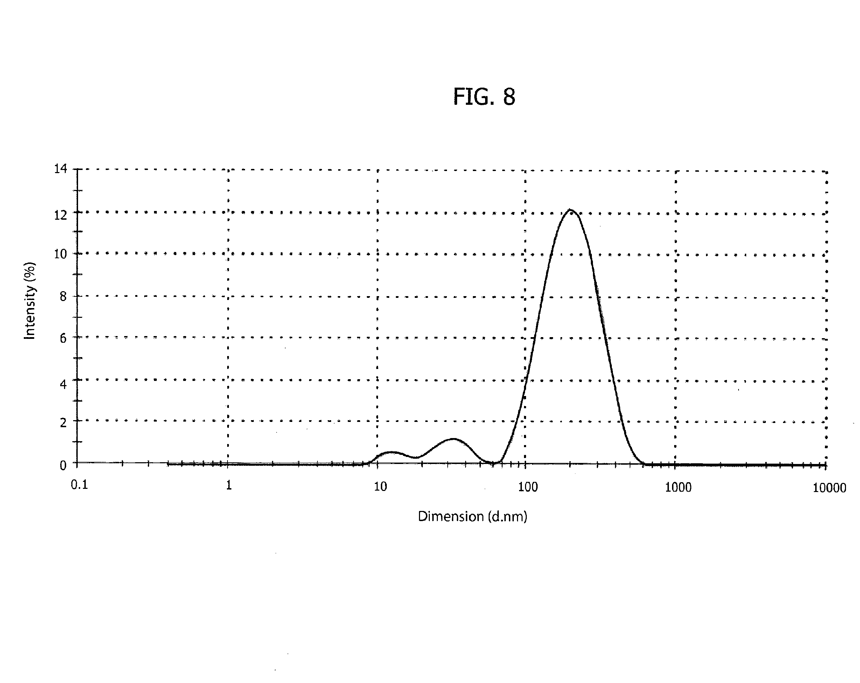

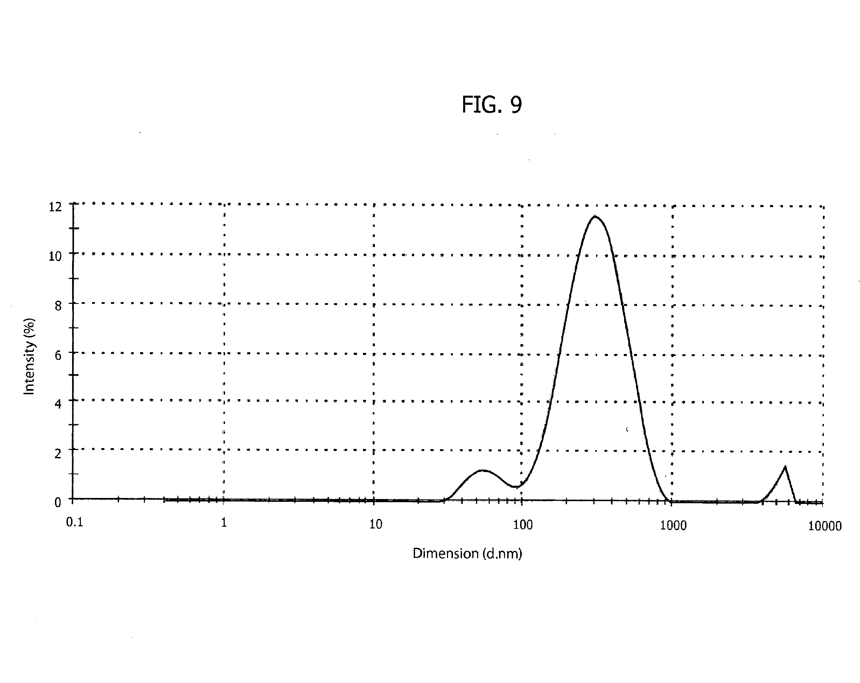

Figures 8, 9 are graphs derived from Light Scattering analysis showing the size distribution of the particles of chitosan incorporating PDRN obtained with solutions 1 and 2 in Table 1.

Table 2 shows the results of the Light Scattering analysis, in which the mean particle diameters (nm) and the relative signal intensity (percentage) are shown.

**Table 2**

| **Sol. 1** | | **Sol. 2** | |
|---|---|---|---|
| **Peaks (nm)** | **Intensity (%)** | **Peaks (nm)** | **Intensity (%)** |
| 210.0 | 91.8 | 179.3 | 90.5 |
| 31.44 | 5.9 | 25.32 | 9.5 |
| 13.56 | 2.3 | 0.000 | 0.0 |

Comparing the first two solutions, both of which have N/P (Chitosan/PDRN) ratios of 4/1, the majority of nanoparticles have a diameter of 210.0 and 179.3 nm, respectively.

Therefore, employing an N/P ratio (chitosan/PDRN) of 4, it is possible to obtain particles with a diameter of about 200 nm.

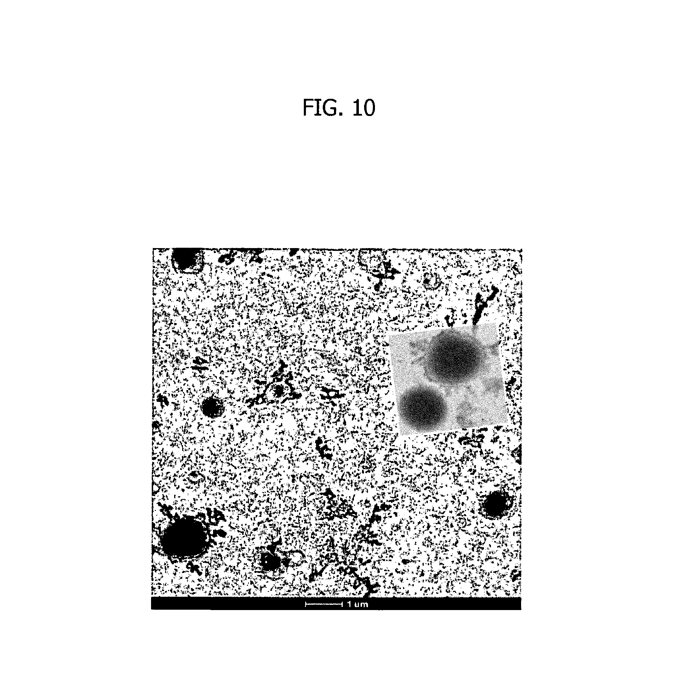

Figure 10 is a transmission electron microscopy image of the nanoparticles obtained with solution 2.

### Production of nanoparticles of chitosan and PDRN through ionic gelation of a (TPP)/PDRN-Na and chitosan system

Nanoparticles of chitosan and PDRN were produced according to the method of ionic gelation (Fan et al., 2012) by adding to a first solution containing chitosan ULWM dissolved in acetic acid a second solution containing 0.5 mg/ml sodium tripolyphosphate (TPP) and 50 µg /ml of PDRN in the form of sodium salt (PDRN-Na) in a 3:10 volume ratio (i.e., 3 ml of solution containing TPP and 10 ml of the solution containing chitosan).

The TTP solution at a temperature of 3-4°C is added quickly to the solution containing chitosan, which had been heated to 60°C for ten minutes, all under continuous mechanical agitation.

Table 3 shows the amount of chitosan, acetic acid, TPP and PDRN in three solutions prepared as described above and subjected to Light Scattering analysis to determine the size of the particles obtained.

**Table 3**

| **Sol.** | **Chitos** | **Acetic Ac.** | **V_{chit}** | **TPP** | **PDRN** | **V_{TPP}** | **N/P** |
|---|---|---|---|---|---|---|---|
| | **mg/ml** | **(%)** | **ml** | **mg/ml** | **µg/ml** | **ml** | - |
| **8** | 0.5 | 0.2 | 10 | 0.5 | 0 | 3 | 3.3/1 |
| **9** | 0.5 | 1 | 10 | 0.5 | 0 | 3 | 3.3/1 |
| **10** | 0.5 | 1 | 10 | 0.5 | 50 | 3 | 3.3/1 |

| | | | | | | | |
|---|---|---|---|---|---|---|---|
| N/P indicates the Chitosan/TTP ratio | | | | | | | |

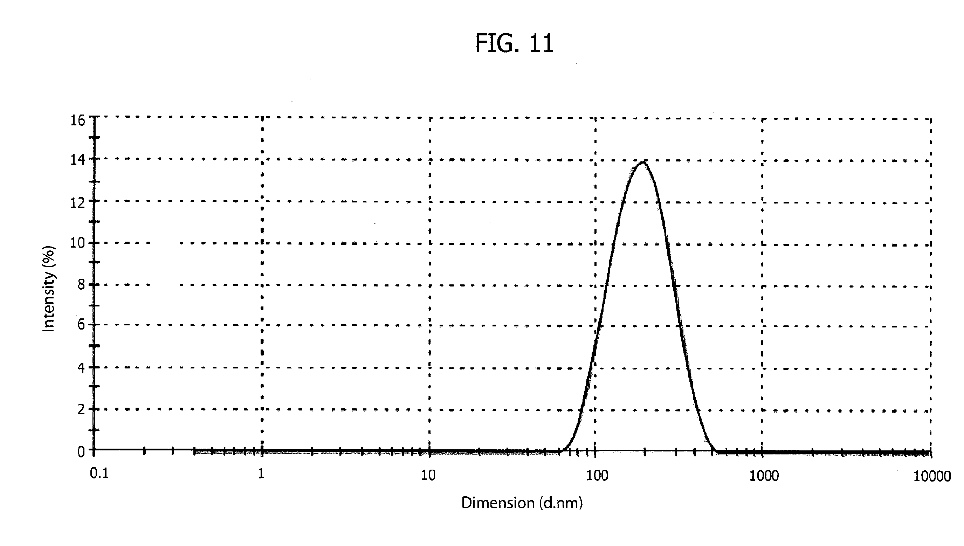

Figure 11 is a graph derived from Light Scattering analysis of solution 10, showing that a monomodal distribution of particle diameters was obtained with a size in the order of 200 nm.

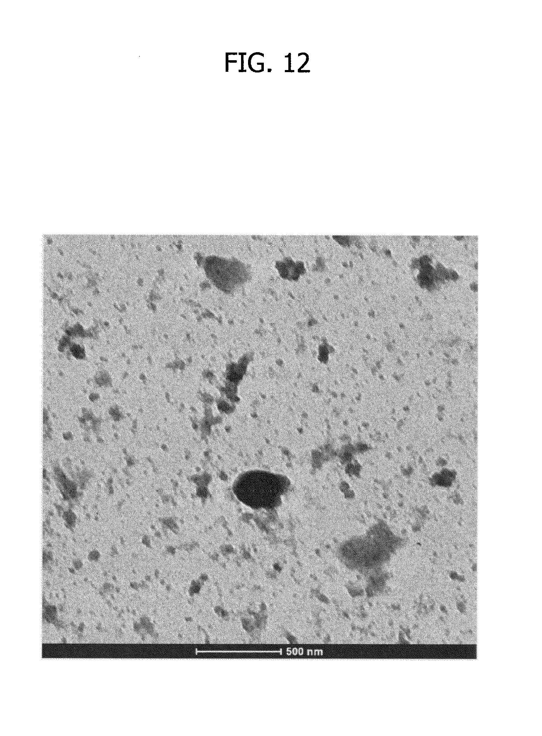

Figure 12 is a transmission electron microscopy image of the nanoparticles obtained with solution 10.

### Analysis of the amount of PDRN incorporated into chitosan nanoparticles

The analysis was performed by UV spectroscopy (λ=260 nm) of the supernatant obtained by centrifugation (Özba 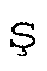 -Turan et al., 2011) at 5000 rpm for 10 minutes of the final solutions obtained as reported above.

Removal of the supernatant and UV spectrophotometric analysis allowed determination of the concentration of unencapsulated nucleic acid (PDRN) remaining in solution.

A calibration curve was prepared with solutions of PDRN/TPP (0.1 mg/ml, 0.04 mg/ml, 0.008 mg/ml and 0.004 mg/ml).

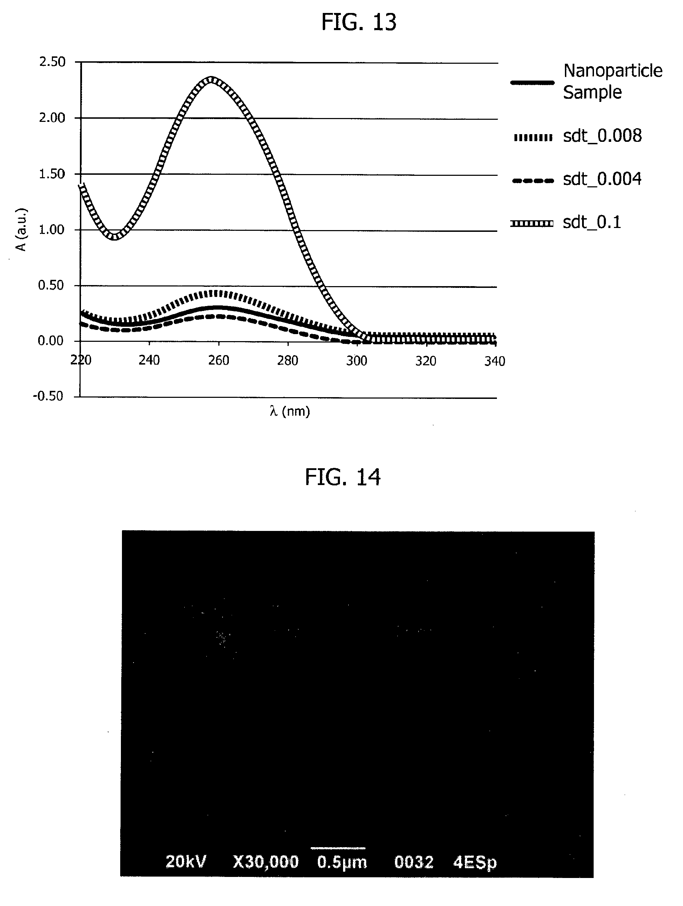

Figure 13 shows the absorption spectrum at 260 nm of the sample of nanoparticles obtained from solution 10 with the ionic gelation method.

In the sample produced from solution 10 by ionic gelation, the chitosan nanoparticles have incorporated about 90% of the PDRN (the initial amount, in the supernatant, was 50 µg/ml, while the amount of PDRN not absorbed, and thus remaining in the supernatant is 4.4 µg/ml).

### Production of carrying structures of PEO-chitosan nanofibers containing chitosan nanoparticles incorporating PDRN

Nanoparticles obtained by ionic gelation were deposited by electrospraying on carrying structures of chitosan 70/1000-PEO.

The following operating conditions were used for electrospraying: voltage between 10 and 20 kV, preferably 12 kV, flow rate between 0 and 3 ml/h, preferably equal to 0.3 ml/h, distance comprised between 10 and 30, preferably equal to 20 cm.

Figure 14 shows a scanning electron microscope image of nanoparticles obtained from solution 9 and deposited by electrospraying.

### Analysis of vitality and adhesion strength of human fibroblasts

To evaluate the viability of cells seeded on chitosan-PEO nanofibers carrying structures comprising chitosan nanoparticles incorporating PDRN described in the previous section of the present description, these carrying structures were anchored to the bottom of wells of cell cultures plates (plates comprising 24-well).

Subsequently, human fibroblasts (200,000 cells/ml; HFF-1, ATCC ® No: SCRC-1041 ™) were deposited in the respective wells.

The same quantity of human fibroblasts was seeded in control wells without carrying structures (CTR-ref 0) or with chitosan-PEO nanofiber carrying structures lacking chitosan nanoparticles incorporating PDRN (CTR-ref 1).

Cell viability was determined after 7 and 14 days of incubation at 37°C, 5% CO₂ by the MTT assay, which employs the compound 3-(4,5-dimethylthiazol-2-yl)-2,5-diphenyl tetrazolium bromide.

The MTT assay is a colorimetric assay that exploits the ability of mitochondrial dehydrogenases in viable cells to cleave the tetrazolium ring of the yellow MTT molecule and produce a violet formazan salt.

The cell viability is determined by reading the optical density (OD) with a spectrophotometer. The number of cells was calculated by extrapolation from a calibration curve prepared in parallel with each assay.

As Tables 4 and 5 show, the wells comprising carrying structures with chitosan nanoparticles incorporating PDRN had a significant increase (p<0.05) in the number of fibroblasts compared to control conditions (CTR-ref 0 and CTR-ref 1) both after 7 days and after 14 days in culture.

**Table 4 - viability test at 7 days**

| | **CTR-ref 0** | **CTR-ref 1** | **carrying str.+ nanoparticles** |
|---|---|---|---|
| | 223385.0 | 220348.3 | 343003.1 |
| | 206718.0 | 233385.3 | 390525.3 |
| **MEDIA** | 215051.5 | 226866.3 | 366764.2 |
| **ST ERROR** | 8333.5 | 6518.5 | 23761.2 |

**Table 5 - viability test at 14 days**

| | **CTR-ref 0** | **CTR-ref 1** | **carrying str.+ nanoparticles** |
|---|---|---|---|
| | 251352.0 | 267014.0 | 356480.7 |
| | 251052.0 | 191385.0 | 433490.8 |
| **MEDIA** | 251202.0 | 229199.5 | 394989.2 |
| **ST ERROR** | 150.0 | 37814.5 | 38501.55 |

Each experimental condition was repeated in duplicate. Data shown represent the mean and standard error of the number of fibroblasts. Statistical analysis was performed using a one-way ANOVA test and Bonferroni post-test using the GraphPad Prism 3.03 program. P-values <0.05 were considered statistically significant.

Furthermore, the capacity of the carrying structures to anchor fibroblasts was evaluated in an analysis that allowed the strength of adhesion of human fibroblasts on these structures to be determined.

The analysis was conducted according to protocols known in the art (Bagno A, et al. Human osteoblast-like cell adhesion on titanium substrates covalently functionalized with synthetic peptides. Bone 2007;40:693-9).

As previously described, chitosan-PEO nanofiber carrying structures comprising chitosan nanoparticles incorporating PDRN were anchored on the bottom of the wells of cell culture plates.

Wells without carrying structures (CTR-ref 0) or with chitosan-PEO nanofiber carrying structures lacking chitosan nanoparticles incorporating PDRN (CTR-ref 1) were used as controls.

Human fibroblasts (200,000 cells/ml) were seeded in the respective wells.

Subsequently, the fibroblasts were incubated with a trypsin-EDTA solution for a period of 20 minutes.

The trypsin-EDTA solution promotes detachment of cells from other cells and from the substrate.

The number of cells that separated from the carrying structures were counted after 7 and 14 days of culture using a Burker counting chamber, whereas the cells that remained adherent to the carrying structures were evaluated by an MTT assay.

Tables 6 and 7 show the number of cells adherent to the substrate after treatment with the trypsin-EDTA solution, measured after 7 and 14 days in culture.

It is evident from Tables 6 and 7 that the force of adhesion of fibroblasts grown on the carrying structure comprising chitosan nanoparticles incorporating PDRN is significantly higher compared to control conditions (CTR-ref 0 and CTR-ref 1).

The wells containing carrying structures with chitosan nanoparticles incorporating PDRN had a significant increase (p<0.05) in the number of fibroblasts compared to control conditions (CTR-ref 0 and CTR-ref 1) both after 7 days and after 14 days in culture.

**Table 6 - viable cells after 7 days in culture**

| | **CTR-ref 0** | **CTR-ref 1** | **carrying str.+ nanoparticles** |
|---|---|---|---|
| | 52.0 | 348.0 | 59258.0 |
| | 52.0 | 52.0 | 61626.0 |
| | 348.0 | 348.0 | 58962.0 |
| **MEDIA** | 150.67 | 249.33 | 59948.67 |
| **ST ERROR** | 98.67 | 98.67 | 843.01 |

**Table 7 - viable cells after 14 days in culture**

| | **CTR-ref 0** | **CTR-ref 1** | **carrying str.+ nanoparticles** |
|---|---|---|---|
| | 52.0 | 348.0 | 75836.0 |
| | 52.0 | 52.0 | 66067.0 |
| | 348.0 | 348.0 | 82644.0 |
| **MEDIA** | 150.67 | 249.33 | 74849.0 |
| **ST ERROR** | 98.67 | 98.67 | 4810.75 |

Each experimental condition was repeated in triplicate. Data shown represent the mean and standard error. Statistical analysis was performed using a one-way ANOVA test and Bonferroni post-test using the GraphPad Prism 3.03 program. P-values <0.05 were considered statistically significant.

### Bibliographic references

Bagno A, et al. Human osteoblast-like cell adhesion on titanium substrates covalently functionalised with synthetic peptides. Bone 2007;40:693-9
Fan W, Yan W, Xu Z, Ni H. Formation mechanism of monodisperse, low molecular weight chitosan nanoparticles by ionic gelation technique. Colloids Surf B Biointerfaces. (2012)90:21-7.
Homayoni H, Hosseini Ravandi SA, Valizadeh M Electrospinning of chitosan nanofibers: processing optimization. Carbohydrate Polymers (2009) 77:656-661.
Mao HQ, Roy K, Troung-Le VL, Janes KA, Lin KY, Wang Y, August JT, Leong KW. Chitosan-DNA nanoparticles as gene carriers: synthesis, characterization and transfection efficiency. J Control Release. (2001) 70(3):399-421.
Mohammadi Z, Abolhassani M, Dorkoosh FA, Hosseinkhani S, Gilani K, Amini T, Najafabadi AR, Tehrani MR. Preparation and evaluation of chitosan-DNA-FAP-B nanoparticles as a novel non-viral vector for gene delivery to the lung epithelial cells. Int J Pharm. (2011) 409(1-2):307-13.
Özba 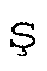 -Turan S, Akbuga J. Plasmid DNA-loaded chitosan/TPP nanoparticles for topical gene delivery. Drug Deliv. (2011) 18 (3) :215-22.

## Claims

1. Medical device (1) comprising nanoparticles (3) comprising chitosan and polydeoxyribonucleotides (PDRN) on a carrying laminar structure (2) comprising chitosan.

2. Medical device (1) according to claim 1, wherein the carrying laminar structure (2) further comprises polyethylene oxide.

3. Medical device (1) according to claim 1, wherein the carrying laminar structure (2) is in the form of nanofibers.

4. Medical device (1) according to claim 3, wherein the nanofibers comprise chitosan.

5. Medical device (1) according to claim 3, wherein the nanofibers comprise chitosan and polyethylene oxide.

6. Medical device (1) according to claim 5, wherein the nanofibers comprise chitosan and polyethylene oxide in a weight ratio between 80:20 and 100:0, preferably equal to 95:5.

7. Medical device (1) according to claim 3, wherein the nanofibers have a diameter between 50 and 200 nm, preferably equal to 100 nm.

8. Medical device (1) according to claim 3, wherein the nanofibers are electrospun nanofibers.

9. Medical device (1) according to claim 3, wherein the nanofibers are cross-linked.

10. Medical device (1) according to any preceding claim, wherein the carrying laminar structure (2) has a thickness between 80-160 µm, preferably about 100-120 µm.

11. Medical device (1) according to any preceding claim, wherein the nanoparticles (3) comprise chitosan and polydeoxyribonucleotides in a weight ratio between 3:1 and 5:1, preferably equal to 4:1.

12. Medical device (1) according to any preceding claim, wherein the nanoparticles (3) have a diameter between 150-250 nm, preferably equal to about 200 nm.

13. Medical device (1) according to any one of the preceding claims, wherein the polydeoxyribonucleotides are present in salt form.

14. Medical device according to claim 13, wherein the salt is selected from at least one of: sodium salt, iron salt, chromium salt, magnesium salt, manganese salt.

## Patentansprüche

1. Medizinische Vorrichtung (1) umfassend Nanopartikel (3) umfassend Chitosan und Polydesoxyribonucleotide (PDRN) auf einer laminaren Trägerstruktur (2) umfassend Chitosan.

2. Medizinische Vorrichtung (1) nach Anspruch 1, wobei die laminare Trägerstruktur (2) außerdem Polyethylenoxid umfasst.

3. Medizinische Vorrichtung (1) nach Anspruch 1, wobei die laminare Trägerstruktur (2) in Form von Nanofasern vorliegt.

4. Medizinische Vorrichtung (1) nach Anspruch 3, wobei die Nanofasern Chitosan umfassen.

5. Medizinische Vorrichtung (1) nach Anspruch 3, wobei die Nanofasern Chitosan und Polyethylenoxid umfassen.

6. Medizinische Vorrichtung (1) nach Anspruch 5, wobei die Nanofasern Chitosan und Polyethylenoxid in einem Gewichtsverhältnis zwischen 80:20 und 100:0, vorzugsweise von 95:5 umfassen.

7. Medizinische Vorrichtung (1) nach Anspruch 3, wobei die Nanofasern einen Durchmesser zwischen 50 und 200 nm, vorzugsweise von 100 nm aufweisen.

8. Medizinische Vorrichtung (1) nach Anspruch 3, wobei die Nanofasern elektrogesponnene Nanofasern sind.

9. Medizinische Vorrichtung (1) nach Anspruch 3, wobei die Nanofasern vernetzt sind.

10. Medizinische Vorrichtung (1) nach einem vorangehenden Anspruch, wobei die laminare Trägerstruktur (2) eine Dicke zwischen 80-160 µm, vorzugsweise ungefähr 100-120 µm aufweist.

11. Medizinische Vorrichtung (1) nach einem vorangehenden Anspruch, wobei die Nanopartikel (3) Chitosan und Polydesoxyribonucleotide in einem Gewichtsverhältnis zwischen 3:1 und 5:1, vorzugsweise von 4:1 umfassen.

12. Medizinische Vorrichtung (1) nach einem vorangehenden Anspruch, wobei die Nanopartikel (3) einen Durchmesser zwischen 150-250 nm, vorzugsweise von ungefähr 200 nm aufweisen.

13. Medizinische Vorrichtung (1) nach einem der vorangehenden Ansprüche, wobei die Polydesoxyribonucleotide in Salzform vorhanden sind.

14. Medizinische Vorrichtung nach Anspruch 13, wobei das Salz ausgewählt ist aus wenigstens einem von: Natriumsalz, Eisensalz, Chromsalz, Magnesiumsalz, Mangansalz.

## Revendications

1. Dispositif médical (1) comprenant des nanoparticules (3) comprenant du chitosane et des polydésoxyribonucléotides (PDRN) sur une structure laminaire porteuse (2) comprenant du chitosane.

2. Dispositif médical (1) selon la revendication 1, dans lequel la structure laminaire porteuse (2) comprend en outre du polyoxyde d'éthylène.

3. Dispositif médical (1) selon la revendication 1, dans lequel la structure laminaire porteuse (2) est sous la forme de nanofibres.

4. Dispositif médical (1) selon la revendication 3, dans lequel les nanofibres comprennent du chitosane.

5. Dispositif médical (1) selon la revendication 3, dans lequel les nanofibres comprennent du chitosane et du polyoxyde d'éthylène.

6. Dispositif médical (1) selon la revendication 5, dans lequel les nanofibres comprennent du chitosane et du polyoxyde d'éthylène dans un rapport pondéral compris entre 80 : 20 et 100 : 0, de préférence égal à 95 : 5.

7. Dispositif médical (1) selon la revendication 3, dans lequel les nanofibres ont un diamètre compris entre 50 et 200 nm, de préférence égal à 100 nm.

8. Dispositif médical (1) selon la revendication 3, dans lequel les nanofibres sont des nanofibres électrofilées.

9. Dispositif médical (1) selon la revendication 3, dans lequel les nanofibres sont réticulées.

10. Dispositif médical (1) selon l'une des revendications précédentes, dans lequel la structure laminaire porteuse (2) a une épaisseur comprise entre 80 et 160 µm, de préférence entre environ 100 et 120 µm.

11. Dispositif médical (1) selon l'une des revendications précédentes, dans lequel les nanoparticules (3) comprennent du chitosane et des polydésoxyribonucléotides dans un rapport pondéral compris entre 3 : 1 et 5 : 1, de préférence égal à 4 : 1.

12. Dispositif médical (1) selon l'une des revendications précédentes, dans lequel les nanoparticules (3) ont un diamètre compris entre 150 et 250 nm, de préférence égal à environ 200 nm.

13. Dispositif médical (1) selon l'une quelconque des revendications précédentes, dans lequel les polydésoxyribonucléotides sont présents sous forme de sel.

14. Dispositif médical selon la revendication 13, dans lequel le sel est choisi parmi au moins l'un : du sel de sodium, du sel de fer, du sel de chrome, du sel de magnésium, du sel de manganèse.
